(19)

**Europäisches Patentamt**
**European Patent Office**
**Office européen des brevets**

(11)  **EP 3 463 657 B1**

(12)  **FASCICULE DE BREVET EUROPEEN**

(45) Date de publication et mention
de la délivrance du brevet:
**26.02.2020  Bulletin 2020/09**

(21) Numéro de dépôt: **17719561.7**

(22) Date de dépôt: **24.04.2017**

(51) Int Cl.:
*B01J 37/03* (2006.01)      *B01J 37/02* (2006.01)
*B01J 35/00* (2006.01)      *B01J 35/10* (2006.01)
*B01J 23/50* (2006.01)      *B01J 23/44* (2006.01)
*B01J 35/02* (2006.01)      *B01J 35/08* (2006.01)
*B01J 37/14* (2006.01)      *B01J 37/18* (2006.01)
*C07C 7/167* (2006.01)      *C07C 7/163* (2006.01)
*C07C 5/05* (2006.01)

(86) Numéro de dépôt international:
**PCT/EP2017/059640**

(87) Numéro de publication internationale:
**WO 2017/207168 (07.12.2017 Gazette 2017/49)**

(54) **PROCEDE DE PREPARATION D'UN CATALYSEUR D'HYDROGENATION SELECTIVE**

VERFAHREN ZUR HERSTELLUNG EINES SELEKTIVEN HYDRIERUNGSKATALYSATORS

METHOD FOR PREPARING A SELECTIVE HYDROGENATION CATALYST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **30.05.2016  FR 1654833**

(43) Date de publication de la demande:
**10.04.2019  Bulletin 2019/15**

(73) Titulaire: **IFP Energies nouvelles**
**92852 Rueil-Malmaison (FR)**

(72) Inventeurs:
* **PETIT-CLAIR, Carine**
  **38200 Jardin (FR)**
* **AVENIER, Priscilla**
  **38000 Grenoble (FR)**
* **MARTIN, Michel**
  **69006 Lyon (FR)**
* **BOUALLEG, Malika**
  **92852 RUEIL-MALMAISON CEDEX (FR)**

(56) Documents cités:
**FR-A1- 2 922 784      FR-A1- 2 968 578**
**FR-A1- 2 991 197      FR-A1- 2 993 795**

EP 3 463 657 B1

**Description**

**Domaine technique**

Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants.

L'objet de l'invention est de proposer un procédé de préparation d'un catalyseur à performances améliorées et un procédé d'hydrogénation sélective des composés hydrocarbonés insaturés présent dans les coupes d'hydrocarbures, de préférence des coupes issues du vapocraquage ou du craquage catalytique.

**Etat de la technique**

Les catalyseurs d'hydrogénation sélective de ces coupes sont souvent à base de palladium, sous forme de petites particules métalliques déposées sur un support qui peut être un oxyde réfractaire. La teneur en palladium et la taille des particules de palladium font partie des critères qui ont une importance sur l'activité et la sélectivité des catalyseurs.

La répartition macroscopique des particules métalliques dans le support constitue également un critère important, principalement dans le cadre de réactions rapides et consécutives telles que les hydrogénations sélectives. Il faut généralement que ces éléments se situent dans une croûte à la périphérie du support afin d'éviter les problèmes de transfert de matière intragranulaire pouvant conduire à des défauts d'activité et une perte de sélectivité. Par exemple, le document US2006/025302 décrit un catalyseur pour l'hydrogénation sélective de l'acétylène et de dioléfines, comprenant du palladium réparti de telle façon que 90% du palladium est introduit dans le catalyseur dans une croûte inférieure à 250 $\mu$m.

Le document FR2922784 divulgue un procédé de préparation d'un catalyseur comprenant du palladium, un support poreux comprenant au moins un oxyde réfractaire sélectionné dans le groupe constitué par la silice, l'alumine et la silice-alumine, dans lequel la surface spécifique du support poreux est comprise entre 50 et 210 m$^2$/g, dans lequel au moins 80 % poids de palladium est réparti dans une croûte à la périphérie du support, l'épaisseur de ladite croûte est comprise entre 20 et 200 $\mu$m, lequel procédé comprend une étape de préparation d'une solution colloïdale d'oxyde de palladium ou d'hydroxyde de palladium en phase aqueuse, une étape d'imprégnation de ladite solution sur le support poreux, une étape de maturation, puis une étape de séchage et calcination. Cependant ce document ne divulgue ni le débit d'imprégnation de la solution sur le support poreux ni le type de dispositif d'imprégnation utilisé.

Le document FR2968578 divulgue un procédé de préparation d'un catalyseur à partir d'une suspension aqueuse colloïdale d'agglomérats de nanoparticules d'oxyde métallique pouvant être imprégné sur un support poreux d'alumine permettant d'obtenir une phase active métallique en périphérie du support. Cependant ce document ne divulgue ni le débit d'imprégnation de la suspension colloïdale sur le support poreux ni le type de dispositif d'imprégnation utilisé.

En général, le dépôt de la phase active de palladium sur le support est réalisé selon toutes les techniques connues de l'homme du métier, par exemple par des techniques d'imprégnation à sec ou par voie humide.

Les catalyseurs d'hydrogénation sélectives sont très souvent mis en forme par des méthodes connues de l'homme du métier, et en particulier par malaxage-extrusion, pastillage, granulation, égouttage dans l'huile (« oil-drop » selon la terminologie anglo-saxonne). Les catalyseurs d'hydrogénation sélective peuvent ainsi se présenter sous formes de billes, cylindres, roue de charrette, cylindre creux, nid d'abeille ou tout autre forme géométrique utilisées par l'homme du métier. Cependant, aucune différenciation n'est apportée sur l'avantage de la mise en forme du support du catalyseur sur la dispersion des particules métalliques sur le support du catalyseur et donc sur son activité catalytique. La mise en forme du catalyseur d'hydrogénation sélective à iso-propriétés structurales et texturales du catalyseur d'hydrogénation sélective n'influe à priori donc pas sur les performances en terme d'activité catalytique.

De manière totalement surprenante, la demanderesse a constaté que des catalyseurs comprenant du palladium et un support poreux de surface spécifique précise préparés à partir d'une technique particulière d'imprégnation du palladium sur ledit support poreux permet d'améliorer la répartition des particules métalliques à la surface dudit catalyseur et permet l'obtention de performances d'activité catalytique améliorées en ce sens que leur activité catalytique est significativement plus importante que celle des catalyseurs obtenus par des méthodes classiques de préparation. Le procédé de préparation selon l'invention permet de préparer des catalyseurs ayant une épaisseur de croûte très fine.

Un catalyseur obtenu par un tel procédé de préparation permet d'offrir une plus grande surface externe disponible et permet une imprégnation plus en surface du support mais également une accessibilité de la charge à la phase active améliorée.

**Objets de l'invention**

La présente invention concerne un procédé de préparation d'un catalyseur comprenant du palladium, un support poreux comprenant au moins un oxyde réfractaire sélectionné dans le groupe constitué par la silice, l'alumine et la silice-alumine, ledit support poreux comprenant une surface spécifique comprise entre 140 et 250 m$^2$/g, dans lequel au moins 80% poids du palladium est réparti dans une croûte à la périphérie dudit support, l'épaisseur de ladite croûte étant comprise entre 20 et 100 $\mu$m, la teneur en palladium dans le catalyseur est comprise entre 0,01 et 2 % poids par rapport au poids total du catalyseur, la dispersion métallique D du palladium est comprise entre 15 % et 70 %, lequel procédé comprenant les étapes suivantes :

a) on prépare une solution colloïdale d'oxyde de palladium ou d'hydroxyde de palladium en phase aqueuse ;
b) on ajoute à un débit compris 1 et 20 litre(s)/heure ladite solution obtenue à l'étape a) sur ledit support poreux ; ledit support poreux étant contenu dans un dispositif d'imprégnation rotatif fonctionnant à une vitesse de rotation comprise entre 4 et 20 tours/minute ;
c) optionnellement, on soumet le support poreux imprégné obtenu à l'étape b) à une maturation afin d'obtenir un précurseur de catalyseur ;
d) on sèche le précurseur de catalyseur obtenu à l'étape b) ou c) à une température comprise entre 70°C et 200°C pendant une durée comprise entre 0,5 et 20 heures ;
e) on calcine le précurseur de catalyseur obtenu à l'étape d) à une température comprise entre 300°C et 500°C.

Dans un mode de réalisation selon l'invention, ledit support poreux se présente sous la forme d'une bille.

Dans un autre mode de réalisation selon l'invention, ledit support poreux se présente sous la forme d'un extrudé.

Avantageusement, ledit support poreux se présente sous la forme d'un extrudé de longueur h comprise entre 2 et 10 mm.

De préférence, ledit support poreux comprend une section comportant au moins trois lobes. Plus préférentiellement, le nombre de lobes de l'extrudé n est choisi dans le groupe constitué par les valeurs entières 3, 4, 5, 6, 7, 8, 9 et 10.

Encore plus préférentiellement, le nombre de lobes n est choisi dans le groupe constitué par les valeurs entières 3 et 4.

Dans un autre mode de réalisations selon l'invention, l'extrudé se présente sous la forme d'un cylindre.

De préférence, ledit catalyseur comprend en outre de l'argent à une teneur comprise entre 0,02 et 3 % poids en argent par rapport au poids total du catalyseur.

Avantageusement, le support présente un diamètre de pores compris entre 2 et 50 nm. Dans un mode de réalisation particulier, on réalise une étape c) de maturation pendant une durée comprise entre 0,5 et 40 heures.

Dans un mode de réalisation particulier, on soumet ledit catalyseur séché obtenu à l'issue de l'étape e) à un traitement réducteur par mise en contact avec un gaz réducteur à une température comprise entre 80 et 180°C.

Avantageusement, le dispositif d'imprégnation rotatif de l'étape b) est un tonneau tournant.

**Description détaillée de l'invention**

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIIIB selon la classification CAS correspond aux métaux des colonnes 8, 9 et 10 selon la nouvelle classification IUPAC.

Les propriétés texturales et structurales du support et du catalyseur décrits ci-après sont déterminées par les méthodes de caractérisation connues de l'homme du métier. Le volume poreux total et la distribution poreuse sont déterminés dans la présente invention par porosimétrie au mercure (cf. Rouquerol F.; Rouquerol J.; Singh K. « Adsorption by Powders & Porous Solids: Principle, methodology and applications », Academic Press, 1999). Plus particulièrement, le volume poreux total est mesuré par porosimétrie au mercure selon la norme ASTM D4284-92 avec un angle de mouillage de 140°, par exemple au moyen d'un appareil modèle Autopore III™ de la marque Micromeritics™. La surface spécifique est déterminée dans la présente invention par la méthode B.E.T, méthode décrite dans le même ouvrage de référence que la porosimétrie au mercure, et plus particulièrement selon la norme ASTM D3663-03.

1. Définitions

Dispersion métallique des particules (D)

La dispersion de particules est un nombre sans unité, souvent exprimé en %. La dispersion est d'autant plus grande que les particules sont petites. Elle est définie dans publication de R. Van Hardeveld et F. Hartog, « The statistics of surface atoms and surface sites on metal crystals», Surface Science 15, 1969, 189-230.

Définition du coefficient R

Les profils de répartition des éléments au sein des grains de catalyseurs sont obtenus par microsonde de Castaing. Au moins 30 points d'analyses sont effectués le long du diamètre de la bille ou de l'extrudé à raison d'une dizaine de points sur la croûte d'un élément actif (ici le palladium) et d'une dizaine de point au centre du grain. Il est ainsi obtenu le profil de répartition $c(x)$ pour $x \in [-r,+r]$ avec c la concentration locale de l'élément, r le rayon de la bille ou de l'extrudé et x la position du point d'analyse le long du diamètre du grain par rapport au centre de ce grain.

La répartition des éléments est caractérisée par un coefficient R adimensionnel pondérant la concentration locale par un poids croissant en fonction de la position sur le diamètre. Par définition :

$$R = \int_{-r}^{r} c(x)x^2 dx \bigg/ \frac{r^2}{3} \int_{-r}^{r} c(x)dx$$

Ainsi un élément dont la concentration est uniforme présente un coefficient R égal à 1, un élément déposé en dôme (concentration au cœur plus importante que la concentration aux bords du support) présente un coefficient supérieur à 1 et un élément réparti en croûte (concentration aux bords plus importante que la concentration au coeur du support) présente un coefficient inférieur à 1. L'analyse par microsonde de Castaing donne les valeurs des concentrations en un nombre fini de valeurs de x, R est ainsi évalué numériquement par des méthodes d'intégration bien connues de l'homme du métier. De préférence, R est déterminé par la méthode des trapèzes.

La répartition de l'élément alcalin est définie comme homogène lorsque le coefficient de répartition R défini ci-dessus est compris entre 0,8 et 1,2.

La répartition de l'élément alcalino-terreux est définie comme homogène lorsque le coefficient de répartition R défini ci-dessus est compris entre 0,8 et 1,2.

Définition de l'épaisseur de croûte de palladium

Afin d'analyser la répartition de la phase métallique sur le support, on mesure une épaisseur de croûte par microsonde de Castaing (ou microanalyse par microsonde électronique). L'appareil utilisé est un CAMECA® XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides.

Lorsque le palladium est reparti en croûte, sa concentration locale diminue généralement progressivement lorsqu'elle est mesurée en partant du bord du grain catalytique vers l'intérieur. Une distance du bord du grain, à laquelle la teneur locale en palladium devient nulle, ne peut souvent pas être déterminée avec précision et reproductibilité. Afin de mesurer une épaisseur de croûte qui est significative pour la majorité des particules de palladium, l'épaisseur de croûte est définie comme la distance au bord du grain contenant 80 % en poids de palladium. Elle est définit dans la publication de L ; Sorbier et al. "Measurement of palladium crust thickness on catalyst by EPMA" Materials Science and Engineering 32 (2012). Pour mesurer une épaisseur de croûte qui est significative pour la majorité des particules de palladium, l'épaisseur de croûte peut de façon alternative être définie comme la distance au bord du grain contenant 80% en poids du palladium. A partir du profil de répartition obtenu par la microsonde de Castaing (c(x)), on peut calculer la quantité cumulée $Q(y)$ de palladium dans le grain en fonction de la distance y au bord du grain de rayon r.

Pour une bille :

$$Q(y) = \int_{-r}^{-y} c(x)4\pi.x^2 dx + \int_{y}^{r} c(x)4\pi.x^2 dx$$

Pour un extrudé :

$$Q(y) = \int_{-r}^{-r+y} c(x) 2\pi.xdx + \int_{r-y}^{r} c(x) 2\pi.xdx$$

avec

    r : rayon du grain ;
    y : distance au bord du grain ;
    x : variable d'intégration (position sur le profil).

On suppose que le profil de concentration suit le diamètre pris de x = - r à x = + r (x = 0 étant le centre).
$Q(r)$ correspond ainsi à la quantité totale de l'élément dans le grain. On résout ensuite numériquement l'équation suivante en y :

$$\frac{Q(y)}{Q(r)} = 0,8$$

c étant une fonction strictement positive, Q est donc une fonction strictement croissante et cette équation possède une seule solution qui est l'épaisseur de croûte.

Rapport de proximité RP

Dans un mode de réalisation selon l'invention, le catalyseur comprend en outre de l'argent (Ag). Les catalyseurs Palladium (Pd) - Argent (Ag) sont caractérisés par microsonde de Castaing. Cette analyse permet de connaitre localement la concentration massique en métal Pd, Ag.
Pour un catalyseur, cette analyse permet de déterminer la répartition relative des deux métaux le long du grain catalytique par intégration d'une succession d'analyses FX à une distance y au bord du grain. La formule permettant d'estimer la proximité des deux métaux est la suivante:
Rapport de proximité =

$$RP(y) = \frac{Q(y)Pd / Q(r)Pd}{Q(y)Ag / Q(r)Ag}$$

avec :

    Q (y) Pd = Somme des concentrations en palladium entre le bord du grain et une distance y du bord du grain catalytique (%pds)
    Q (y) Ag = Somme des concentrations en argent entre le bord du grain et une distance y du bord du grain catalytique (%pds)
    Q (r) Pd = teneur en palladium totale du grain catalytique (%pds)
    Q (r) Ag= teneur en argent totale du grain catalytique (%pds).

On définit ainsi un critère de proximité, qui rend compte de la localisation relative des deux métaux dans le support. Ce dernier, déterminé par microsonde représente le rapport massique en tout point y du support des éléments métalliques ajoutés, dans notre cas Pd et Ag. Le rapport de proximité d'un catalyseur contant des métaux localement uniformément répartis sera de 1.
Dans le catalyseur selon l'invention, le rapport de proximité RP est compris entre 0,5 et 2, de préférence entre 0,8 et 1,4.

1. Procédé de préparation du catalyseur

Le catalyseur préparé par le procédé de préparation selon l'invention est un catalyseur comprend du palladium, un support un support poreux comprenant au moins un oxyde réfractaire sélectionné dans le groupe constitué par la silice, l'alumine et la silice-alumine, ledit support poreux comprenant une surface spécifique comprise entre 140 et 250

m$^2$/g, dans lequel au moins 80% poids du palladium le palladium est réparti dans une croûte à la périphérie dudit support, l'épaisseur de ladite croûte étant comprise entre 20 et 100 $\mu$m, de préférence entre 25 et 90 $\mu$m, la teneur en palladium dans le catalyseur est comprise entre 0,01 et 2 % poids par rapport au poids total du catalyseur, la dispersion métallique D du palladium est comprise entre 15 % et 70 %, ledit catalyseur étant préparé par un procédé comprenant au moins les étapes suivantes :

a) on prépare une solution colloïdale d'oxyde de palladium ou d'hydroxyde de palladium en phase aqueuse ;
b) on ajoute à un débit compris 1 et 20 litre(s)/heure ladite solution obtenue à l'étape a) sur ledit support poreux ; ledit support poreux étant contenu dans un dispositif d'imprégnation rotatif fonctionnant à une vitesse de rotation comprise entre 4 et 20 tours/minute ;
c) optionnellement, on soumet le support poreux imprégné obtenu à l'étape b) à une maturation afin d'obtenir un précurseur de catalyseur ;
d) on sèche le précurseur de catalyseur obtenu à l'étape b) ou c) à une température comprise entre 70°C et 200°C pendant une durée comprise entre 0,5 et 20 heures ;
e) on calcine le précurseur de catalyseur obtenu à l'étape d) à une température comprise entre 300°C et 500°C.

Les différentes étapes du procédé menant au catalyseur selon l'invention seront détaillées par la suite.

a) préparation d'une suspension colloïdale d'oxyde de palladium ou d'hydroxyde de palladium en phase aqueuse

La suspension colloïdale est généralement obtenue par hydrolyse du cation palladium en milieu aqueux, ce qui conduit à la formation de particules d'oxyde ou d'hydroxyde de palladium en suspension.
La solution aqueuse d'hydroxyde d'alcalins ou d'hydroxyde alcalino-terreux est généralement sélectionnée dans le groupe constitué par les solutions aqueuses d'hydroxyde de sodium, les solutions aqueuses d'hydroxyde de magnésium. De manière préférée, de préférence la solution aqueuse est une solution aqueuse d'hydroxyde de sodium.
Typiquement, on approvisionne dans un appareillage adapté la solution aqueuse comprenant au moins un sel précurseur du palladium [appelée aussi ici solution (II)] puis la solution aqueuse comprenant au moins un hydroxyde d'alcalins ou d'alcalino-terreux [appelée aussi ici solution (I)]. Alternativement, les solutions (I) et (II) peuvent être versées simultanément dans l'appareillage. De préférence, la solution aqueuse (II) puis la solution aqueuse (I) est versée dans l'appareillage.
Le sel précurseur du palladium est généralement sélectionné dans le groupe constitué par le chlorure de palladium, le nitrate de palladium et le sulfate de palladium. De manière très préférée, le sel précurseur du palladium est le nitrate de palladium.
La suspension colloïdale reste généralement dans l'appareillage pendant un temps de séjour compris entre 0 et 20 heures.
Les concentrations de la solution (I) et (II) sont généralement choisies afin d'obtenir un pH de la suspension colloïdale compris entre 1,0 et 3,5. Ainsi, le pH de la suspension colloïdale peut être modifié pendant ce temps de séjour par ajout de quantités d'acide ou de base compatibles avec la stabilité de la suspension colloïdale.
En général, la température de préparation est comprise entre 5°C et 40°C et de manière préférée entre 15°C et 35°C.
La concentration en palladium est de préférence comprise entre 5 et 150 millimoles par litre (mmol/L), de manière plus préférée entre 8 et 80 millimoles par litre.

b) dépôt de la suspension colloïdale préparée à l'étape a) par imprégnation sur un support.

La suspension colloïdale préparée à l'étape a) est ensuite imprégnée sur un support poreux. Le support poreux comprend au moins un oxyde réfractaire sélectionné dans le groupe constitué par la silice, l'alumine et la silice-alumine. De préférence, le support poreux est de l'alumine.
Le support poreux peut se présenter soit sous la forme d'une bille, soit sous la forme d'un extrudé. On entend par un support extrudé, un support comprenant une longueur h et un diamètre équivalent Déq dans lequel la longueur h est supérieure au diamètre équivalent Déq. De préférence l'extrudé comprend une longueur h comprise entre 2 et 10 mm, de préférence entre 2 et 8 mm, et plus préférentiellement entre préférence entre 3 et 6 mm.
De préférence, l'extrudé est sélectionné parmi les cylindres et les extrudés comprenant une section comportant au moins trois lobes.
Dans un mode de réalisation préféré selon l'invention, l'extrudé comprend une section comportant au moins trois lobes.
Lorsque l'extrudé comprend une section comportant au moins trois lobes, la section de l'extrudé peut être caractérisée par un rayon R répondant à l'équation (1):

$$R = \cos\theta \cdot (R_o - r) + \sqrt{\cos^2\theta \cdot (R_o - r)^2 - R_o \cdot (R_o - 2 \cdot r)}$$

$$\text{avec} \quad \theta = \alpha - k \cdot \frac{2 \cdot \pi}{n} \qquad et \qquad k = Int\left(\frac{\left|\alpha + \frac{\pi}{2}\right|}{\frac{2 \cdot \pi}{n}}\right)$$

$$et\, \alpha \in [0\,,\, 2\pi] \tag{1}$$

où $R_o$ représente la distance maximale entre le centre de l'extrudé et la paroi de l'extrudé, R représente la distance entre le centre de l'extrudé et la paroi de l'extrudé pour un angle a, r représente le rayon d'un lobe de l'extrudé, n correspond au nombre de lobes de l'extrudé, la fonction Int() représente la partie entière du rapport $\left(\dfrac{\left|\alpha + \frac{\pi}{2}\right|}{\frac{2 \cdot \pi}{n}}\right)$ et

$\left|\alpha + \dfrac{\pi}{2}\right|$ représente la valeur absolue de la somme $\alpha + \dfrac{\pi}{2}$.

Selon la présente invention, on entend par la fonction Int(), la partie entière du rapport $\left(\dfrac{\left|\alpha + \frac{\pi}{2}\right|}{\frac{2 \cdot \pi}{n}}\right)$.

Ainsi, de manière illustrative, l'application de la fonction Int () pour un rapport égal à 1,8 correspond à la valeur entière 1 c'est à dire Int(1,8)=1, et l'application de la fonction Int () pour un rapport égal à 2,1 correspond à la valeur entière 2 c'est à dire Int(2,1)=2.

Avantageusement, dans ce mode particulier selon l'invention, le nombre de lobes de l'extrudé n est choisi dans le groupe constitué par les valeurs entières 3, 4, 5, 6, 7, 8, 9 et 10; de manière préférée le nombre de lobes n est choisi dans le groupe constitué par les valeurs entières 3, 4, 5 et 6; de manière plus préférée, le nombre de lobes n est choisi dans le groupe constitué par les valeurs entières 3 et 4; et de manière très préférée le nombre de lobes n est 3.

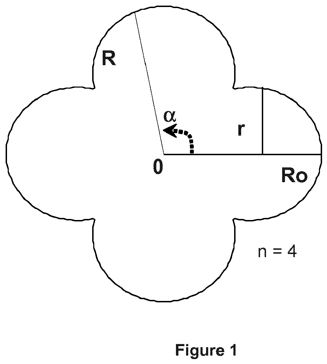

Pour plus de clarté dans l'application de l'équation (1) selon l'invention, la figure 1 montre un schéma illustratif et non limitatif d'une section d'un extrudé dans lequel sont représentés tous les paramètres $R_o$, R, r et a, n étant le nombre de lobes de l'extrudé. La section de l'extrudé correspond à une coupe de l'extrudé selon un plan perpendiculaire à la direction de l'extrusion. En référence à la figure 1, la section de l'extrudé comporte quatre lobes.

Les procédés de fabrication d'extrudés supportés connus de l'homme du métier engendrent souvent des imperfections de forme liées à la mécanique des phases en présence, ce qui peut engendrer un écart entre la valeur R mesurable ($R_{mes}$) et la valeur R définie par l'équation (1). La valeur R mesurable ($R_{mes}$) liée à la valeur R définie par l'équation (1) de la présente invention est avantageusement comprise entre R-15%R et R+15%R, de préférence entre R-10%R et R+10%R, de manière plus préférée entre R-5%R et R+5%R, de manière encore plus préférée entre R-3%R et R+3%R.

Selon l'invention, la surface spécifique du support poreux est comprise entre 140 et 250 $m^2/g$, de préférence entre 165 et 250 $m^2/g$, plus préférentiellement entre 170 et 220 $m^2/g$ et encore plus préférentiellement entre 175 et 210 $m^2/g$.

Le volume poreux du support est généralement compris entre 0,1 et 1,5 $cm^3/g$, de préférence compris entre 0,2 et 1,0 $cm^3/g$.

De préférence, le support du catalyseur d'hydrogénation sélective est purement mésoporeux, i.e. qu'il présente un diamètre de pores compris entre 2 et 50 nm, de préférence entre 5 et 30 nm et de manière encore préférée de 8 à 20 nm. Dans ce mode de réalisation, le support de catalyseur ne comprend donc ni de micropores (< 2 nm), ni de macropores (>50 nm).

Le support peut comprendre éventuellement du soufre. Le soufre peut provenir d'au moins un des précurseurs de synthèse du support d'alumine, notamment le sulfate d'aluminium. Selon le pH de la précipitation du gel d'alumine, une quantité de soufre résiduelle est contenue dans le support final. La teneur en soufre comprise dans le support peut être comprise entre 0,0050 et 0,25 % en poids par rapport au poids total du catalyseur, de préférence entre 0,0075 et 0,20 % en poids.

Le support peut éventuellement subir un ensemble de traitements avant l'étape d'imprégnation, tel que des calcinations ou des hydratations. Le support peut aussi déjà comprendre un ou plusieurs éléments métalliques avant l'imprégnation de la suspension colloïdale. Des éléments métalliques peuvent aussi être introduits dans la suspension colloïdale. Ces éléments métalliques peuvent être introduits soit par des techniques conventionnelles, soit en utilisant le procédé selon la présente invention.

Selon l'invention, la suspension colloïdale est versée sur le support. Ce processus est réalisé dans un dispositif d'imprégnation rotatif, tel qu'un tonneau imprégnateur, de façon continue, c'est-à-dire que l'étape de préparation de la suspension colloïdale précède l'étape d'imprégnation sur le support et que l'essentiel de la suspension colloïdale est envoyée vers l'étape d'imprégnation, en continu, après ajustement du temps de séjour de la suspension colloïdale à l'étape b).

Le dispositif d'imprégnation rotatif utilisé est de préférence un tonneau imprégnateur classique dont l'enceinte peut être mise sous pression réduite (environ 20 mm Hg) ou sous balayage de gaz (azote).

Le dispositif d'imprégnation rotatif est équipé d'une double-enveloppe, dans laquelle circule un fluide caloporteur via un thermorégulateur. Il est ainsi possible de réguler une température de paroi au sein de l'imprégnateur et un temps de séchage. Dans un mode de réalisation préféré, la température d'imprégnation est comprise entre 40 et 90°C, de préférence entre 50 et 70°C.

Selon l'invention, le dispositif d'imprégnation rotatif dans lequel le support, de surface spécifique comprise entre 140 et 250 m$^2$/g, a été chargé fonctionne à une vitesse de rotation comprise entre 4 et 20 tours/minute. Au-dessus de 20 tours/minute la croûte de palladium obtenue sur le support est trop faible, i.e. en dessous de 20 $\mu$m, et une partie du palladium n'est pas imprégnée sur le support. Si la rotation du tambour est trop faible, i.e. inférieure à 4 tours/minute, la croûte de palladium obtenue sur le support peut excéder 100 $\mu$m d'épaisseur, et la dispersion du palladium sur le support n'est pas satisfaisante, c'est à dire inférieure à 15%.

Selon l'invention, le débit d'ajout de la solution colloïdale obtenue à l'étape a) sur le support poreux est compris entre 1 et 20 litre(s) par heure. Au-dessus de 20 litres par heure, la croûte de palladium obtenue est trop épaisse, i.e. au-dessus de 100 $\mu$m et la dispersion du palladium n'est pas satisfaisante, c'est à dire inférieure à 15%.

On peut par exemple citer comme processus continu, un processus où la solution colloïdale est versée dans un bac qui se déverse en continu dans un tambour tournant comprenant le support à imprégner.

c) maturation du support imprégné lors de l'étape b) pendant une durée comprise entre 0,5 et 40 heures (étape optionnelle)

Après imprégnation, le support imprégné peut être maturé à l'état humide pendant 0,5 à 40 h, de manière préférée pendant 1 à 30 h.

d) séchage du précurseur de catalyseur obtenu à l'étape b) ou c)

Le précurseur du catalyseur est généralement séché afin d'éliminer toute ou une partie de l'eau introduite lors de l'imprégnation, de préférence à une température comprise entre 50°C et 250°C, de manière plus préférée entre 70°C et 200°C. La durée du séchage est comprise entre 0,5 h et 20 h.

Le séchage est généralement effectué sous air de combustion d'un hydrocarbure, de préférence du méthane, ou sous air chauffé comprenant entre 0 et 80 grammes d'eau par kilogramme d'air de combustion, un taux d'oxygène compris entre 5% et 25% volume et un taux de dioxyde de carbone compris entre 0% et 10% volume.

e) calcination sous air de combustion du catalyseur séché obtenu à l'étape d)

Après séchage le catalyseur est généralement calciné sous air de combustion, de préférence un air de combustion du méthane comprenant entre 40 et 80 gramme d'eau par kg d'air de combustion, un taux d'oxygène compris entre 5% et 15% volume et un taux de $CO_2$ compris entre 4% et 10% volume. La température de calcination est généralement comprise entre 250°C et 900°C, de préférence comprise entre environ 300°C et environ 500°C. La durée de calcination est généralement comprise entre 0,5 h et 5 h.

f) réduction de l'oxyde ainsi supporté obtenu à l'étape e), de préférence au moyen d'hydrogène gazeux (étape optionnelle)

Le catalyseur est généralement réduit. Cette étape est de préférence réalisée en présence d'un gaz réducteur, soit in-situ, c'est-à-dire dans le réacteur où est réalisée la transformation catalytique, soit ex-situ. De manière préférée, cette étape est effectuée à une température comprise entre 80°C et 180°C, de manière encore plus préférée entre 100°C et 160°C.

La réduction est réalisée en présence d'un gaz réducteur comprenant entre 25 vol% et 100 vol% d'hydrogène, de préférence 100% volume d'hydrogène. L'hydrogène est éventuellement complété par un gaz inerte pour la réduction, de préférence de l'argon, de l'azote ou du méthane.

La réduction comprend généralement une phase de montée en température puis un palier. La durée du palier de réduction est généralement comprise entre 1 et 10 heures, de préférence entre 2 et 8 heures.

La Vitesse Volumétrique Horaire (V.V.H) est généralement comprise entre 150 et 3000, de préférence entre 300 et 1500 litres de gaz réducteur par heure et par litre de catalyseur.

Selon une variante, le catalyseur peut contenir un ou plusieurs métaux promoteurs, en particulier de l'argent. Le ou les métaux promoteurs peuvent être introduit lors de la préparation du support, sur le support déjà formé, pendant l'étape a) ou à l'issue des étapes b), c), d), e) ou f).

Dans un mode de réalisation particulier selon l'invention, le catalyseur comprend en outre de l'argent. L'argent peut être introduit lors de la préparation du support, sur le support déjà formé, pendant l'étape a) ou à l'issue des étapes b), c), d), e) ou f).

Le dépôt d'argent sur le support peut avantageusement être réalisé par toute méthode connue de l'homme du métier, préférentiellement par imprégnation dudit support par au moins une solution contenant au moins un précurseur d'argent, et de préférence, par imprégnation à sec ou par imprégnation en excès. Cette solution contient au moins un précurseur d'argent à la concentration voulue pour obtenir sur le catalyseur final une teneur en argent comprise entre 0,02 et 3 % poids en argent par rapport au poids total du catalyseur, de préférence entre 0,05 et 0,3 % poids.

Dans le mode de réalisation dans lequel le catalyseur comprend en outre de l'argent, au moins 80 % poids de l'argent étant réparti dans une croûte à la périphérie du support, l'épaisseur de ladite croûte étant comprise entre 20 et 100 $\mu$m, de préférence comprise entre 25 et 90 $\mu$m, la teneur locale en argent à chaque point le long du diamètre du grain ayant la même évolution que la teneur locale en palladium.

Le catalyseur obtenu par le procédé selon l'invention peut comprendre au moins un métal sélectionné dans le groupe constitué par les alcalins et les alcalino-terreux.

Le métal alcalin est généralement sélectionné dans le groupe constitué par le lithium, le sodium, le potassium, le rubidium et le césium, de préférence par le lithium, le sodium et le potassium, de manière très préférée par le sodium et le potassium. De manière encore plus préférée, le métal alcalin est le sodium.

Le métal alcalino-terreux est généralement sélectionné dans le groupe constitué par le magnésium, le calcium, le strontium et le baryum, de préférence par le magnésium et le calcium, de manière très préférée par le magnésium.

Le métal alcalin, lorsqu'il est présent, est réparti de manière homogène à travers le support avec un coefficient R compris entre 0,8 et 1,2.

Le métal alcalino-terreux, lorsqu'il est présent, est réparti de manière homogène à travers le support avec un coefficient R compris entre 0,8 et 1,2.

Le catalyseur obtenu par le procédé selon l'invention comprend une dispersion métallique (D) d est comprise entre 15% et 70%, de préférence entre 20% et 70%, et plus préférentiellement entre 25% et 60%.

Le catalyseur obtenu par le procédé selon l'invention comprend une teneur en palladium comprise entre 0,01 et 2 % poids en palladium, de façon préférée entre 0,05 et 1% poids, par rapport au poids total du catalyseur.

Utilisation du catalyseur

Le catalyseur selon l'invention peut être utilisé dans les procédés faisant intervenir une transformation de composés organiques. Ainsi, le catalyseur selon l'invention peut être utilisé dans les procédés comprenant des réactions d'hydrogénation des composés comportant des fonctions aromatiques, cétones, aldéhydes, acides ou nitro, l'hydrogénation du monoxyde de carbone en alcools C1-C6, en méthanol ou en diméthyl-éther, les réactions d'isomérisation ou d'hydro-isomérisation, d'hydrogénolyse, et d'une manière générale les réactions faisant intervenir des coupures ou des formations de liaisons carbone-carbone.

Les conditions opératoires généralement utilisées pour ces réactions sont les suivantes: une température comprise entre 0°C et 500°C, de préférence entre 25 et 350°C, une pression comprise entre 0,1 et 20 MPa, de préférence entre 0,1 et 10 MPa, une vitesse volumique horaire (V.V.H.) comprise entre 0,1 et 50 h$^{-1}$, de préférence entre 0,5 et 20 h$^{-1}$ pour une charge liquide; et entre 500 et 30 000 h$^{-1}$, de préférence entre 500 et 15 000 h$^{-1}$ pour une charge gazeuse. Lorsque de l'hydrogène est présent, le rapport molaire hydrogène sur charge est compris entre 1 et 500 litres par litre, de préférence entre 10 et 150 litres par litre.

La mise en œuvre du catalyseur selon l'invention et les conditions de son utilisation doivent être adaptées par l'utilisateur à la réaction et à la technologie utilisée.

Le catalyseur selon l'invention peut aussi être utilisé dans les réactions d'hydrogénation des composés comportant des fonctions acétyléniques, diéniques, oléfiniques.

L'invention concerne aussi le procédé d'hydrogénation sélective par mise en contact d'une charge sur le catalyseur selon l'invention ou sur le catalyseur préparé selon l'invention, ladite charge étant sélectionnée dans le groupe constitué par les coupes C3 de vapocraquage, les coupes C4 de vapocraquage, les coupes C5 de vapocraquage et les essences de vapocraquage appelée aussi essences de pyrolyse.

Selon une application préférée, les catalyseurs selon l'invention sont mise en oeuvre pour les réactions d'hydrogénation sélective des coupes poly-insaturées d'hydrocarbures issues de vapocraquage et/ou de craquage catalytique, de préférence des coupes poly-insaturées d'hydrocarbures issues du vapocraquage.

L'hydrogénation des coupes C3 à C5

Les procédés de conversion des hydrocarbures tels que le vapocraquage ou le craquage catalytique sont opérés à haute température et produisent une grande variété de molécules insaturées telles que l'éthylène, le propylène, les butènes linéaires, l'isobutène, les pentènes ainsi que des molécules insaturés contenant jusqu'à environ 15 atomes de carbone.

En parallèle sont également formés des composés polyinsaturés: acétylène, propadiène et méthylacétylène (ou propyne), 1-2 et 1-3 butadiène, vinylacétylène et éthylacétylène, et d'autres composés polyinsaturés dont le point d'ébullition correspond à la fraction essence C5+.

Tous ces composés polyinsaturés doivent être éliminés pour permettre l'utilisation de ces différentes coupes dans les procédés de pétrochimie tels que les unités de polymérisation.

Ainsi, par exemple, la coupe C3 de vapocraquage peut avoir la composition moyenne suivante : de l'ordre de 90% poids propylène, de l'ordre de 3 à 8% poids de propadiène et méthylacétylène, le reste étant essentiellement du propane. Dans certaines coupes C3, entre 0,1 et 2% poids de C2 et de C4 peut aussi être présent. Les spécifications concernant les concentrations de ces composés polyinsaturés pour les unités de pétrochimie et de polymérisation sont très basses : 20-30 ppm poids de MAPD (MéthylAcétylène et PropaDiène) pour le propylène qualité chimique et moins de 10 ppm poids voire jusqu'à 1 ppm poids pour la qualité « polymérisation ».

Une coupe C4 de vapocraquage présente par exemple la composition molaire moyenne suivante : 1 % de butane, 46,5% de butène, 51% de butadiène, 1,3% de VinylAcétylène (VAC) et 0,2% de butyne. Dans certaines coupes C4, entre 0,1 et 2% poids de C3 et de C5 peut aussi être présent. Là encore les spécifications sont sévères : teneur en dioléfines strictement inférieure à 10 ppm poids pour une coupe C4 qui sera utilisée en pétrochimie ou polymérisation.

Une coupe C5 de vapocraquage présente par exemple la composition moyenne en masse suivante : 21% de pentanes, 45% de pentènes, 34% de pentadiènes.

Le procédé d'hydrogénation sélective s'est progressivement imposé pour éliminer les composés polyinsaturés des coupes pétrolières C3 à C5 citées car ce procédé permet la conversion des composés les plus insaturés vers les alcènes correspondants en évitant la saturation totale et donc la formation des alcanes correspondant.

L'hydrogénation sélective peut être réalisée en phase gaz ou liquide, de préférence en phase liquide. En effet, une réaction en phase liquide permet d'abaisser le coût énergétique et d'augmenter la durée de cycle des catalyseurs. Pour une réaction en phase liquide, la pression est généralement comprise entre 1 et 3 MPa, la température entre 2 et 50°C et le ratio molaire hydrogène/(composés polyinsaturés à hydrogéner) entre 0,1 et 4, de préférence entre 1 et 2.

Pour une réaction d'hydrogénation en phase gazeuse, la pression est généralement comprise entre 1 et 3 MPa, la température entre 40 et 120°C et le ratio molaire hydrogène/(composés polyinsaturés à hydrogéner) entre 0,1 et 4, de préférence entre 1 et 2.

L'hydrogénation des essences de vapocraquage

Le vapocraquage produit principalement de l'éthylène, du propylène, une coupe C4 ainsi que de l'essence de vapocraquage appelée aussi essence de pyrolyse.

Selon un autre mode préféré, la charge est une essence de pyrolyse. L'essence de pyrolyse correspond à une coupe dont la température d'ébullition est généralement comprise entre 0°C et 250°C, de préférence entre 10°C et 220°C. Cette charge comprend généralement la coupe C5-C12 avec des traces de C3, C4, C13, C14, C15 (par exemple entre 0,1 à 3% poids pour chacune de ces coupes).

Par exemple, une coupe C5-200°C a généralement une composition en % poids suivante:

Paraffines 8 - 12 ;
Aromatiques 58 - 62 ;

Mono-oléfines 8 - 10 ;
Dioléfines 18-22 ;
Soufre 20 - 300 ppm.

L'hydrogénation sélective d'une essence de pyrolyse consiste à mettre en contact la charge à traiter avec de l'hydrogène introduit en excès dans un ou plusieurs réacteurs contenant le catalyseur d'hydrogénation.

Le débit d'hydrogène est ajusté afin d'en disposer en quantité suffisante pour hydrogéner théoriquement l'ensemble des dioléfines, des acétyléniques et des alkényl aromatiques et de maintenir un excès d'hydrogène en sortie de réacteur.

Afin de limiter le gradient de température dans le réacteur, il peut être avantageux de recycler une fraction de l'effluent à l'entrée et/ou au milieu du réacteur.

Dans le cas d'une hydrogénation sélective d'essence de pyrolyse, le ratio molaire hydrogène/(composés polyinsaturés à hydrogéner) est généralement compris entre 1 et 2, la température est généralement comprise entre 40°C et 200°C, de préférence entre 50 et 180°C, la vitesse horaire spatiale (correspondant au volume d'hydrocarbure par volume de catalyseur et par heure) est comprise généralement entre 0,5 h-1 et 10 h-1 , de préférence entre 1 h-1 et 5 h-1 et la pression est généralement comprise entre 1,0 MPa et 6,5 MPa, de préférence entre 2,0 MPa et 3,5 MPa.

## EXEMPLES

Les exemples présentés ci-dessous visent à démontrer l'amélioration de l'activité catalytique en hydrogénation sélective des catalyseurs selon l'invention. Les exemples 1 à 3 et 8 concernent des procédés de préparation de catalyseurs non conformes à l'invention et les exemples 4 à 7 concernent des procédés de préparation d'un catalyseur selon l'invention. L'exemple 9 concerne l'application de ces catalyseurs dans une réaction d'hydrogénation sélective.

### Exemple 1 : préparation d'un catalyseur C1 (non conforme)

Dans cet exemple, la surface spécifique du support du catalyseur est inférieure à celle des catalyseurs conformes à l'invention (i.e. inférieure à 140 m$^2$/g). Par ailleurs, l'étape d'imprégnation n'est pas conforme à l'invention.

Une suspension colloïdale d'oxyde de Pd est préparée sous agitation à 25°C par dilution de 230 g d'une solution de nitrate de palladium Pd(NO$_3$)$_2$ contenant 8,5 % poids de palladium Pd avec environ 4,8L d'eau déminéralisée, puis ajout d'environ 150ml d'une solution de soude pour arriver à un pH de 2,4. La suspension est ensuite diluée avec de l'eau déminéralisée à un volume qui correspond au volume poreux du support alumine dont la surface spécifique est de 71 m$^2$/g mise en forme sous forme de billes. Cette solution est ensuite imprégnée en tonneau d'imprégnation à une vitesse de rotation de 6 tours/minute (tr/min) et un débit d'ajout de la solution de 22 lites/heure (l/h) sur environ 10 kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C1 ainsi préparé contient 0,19% poids de palladium par rapport au poids total de catalyseur.

La caractérisation du catalyseur C1 par microsonde de Castaing montre que 80% du Pd est réparti sur une croûte d'épaisseur environ 189 $\mu$m.

Le sodium est réparti de façon homogène avec un coefficient de répartition R(Na)=0,92.

La dispersion apparente du palladium du catalyseur C1 est de 22%.

### Exemple 2 : préparation d'un catalyseur C2 (non conforme)

Dans cet exemple, l'étape d'imprégnation n'est pas conforme à l'invention.

Une suspension colloïdale d'oxyde de Pd est préparée sous agitation à 25°C par dilution de 230 g d'une solution de nitrate de palladium Pd(NO$_3$)$_2$ contenant 8,5 % poids de palladium Pd avec environ 7,5L d'eau déminéralisée, puis ajout d'environ 150ml d'une solution de soude pour arriver à un pH de 2,4. La suspension est ensuite diluée avec de l'eau déminéralisée à un volume qui correspond au volume poreux du support alumine dont la surface spécifique est de 140 m$^2$/g mise en forme sous forme de billes. Cette solution est ensuite imprégnée en tonneau d'imprégnation à une vitesse de rotation de 6 tr/min et un débit d'ajout de la solution de 22 l/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C2 ainsi préparé contient 0,19% poids de palladium par rapport au poids total de catalyseur.

La caractérisation du catalyseur C2 par microsonde de Castaing montre que 80% du Pd est réparti sur une croûte d'épaisseur environ 150 $\mu$m.

Le sodium est réparti de façon homogène avec un coefficient de répartition R(Na)=0,92. La dispersion apparente du palladium du catalyseur C2 est de 26%.

### Exemple 3 : préparation d'un catalyseur C3 (non conforme)

Dans cet exemple, la surface spécifique du support est inférieure à celle des catalyseurs conformes à l'invention (i.e. inférieure à 140 m$^2$/g). Cependant, l'étape d'imprégnation est conforme à l'invention.

Une suspension colloïdale d'oxyde de Pd est préparée sous agitation à 25°C par dilution de 230 g d'une solution de nitrate de palladium Pd(NO$_3$)$_2$ contenant 8,5 % poids de palladium Pd avec environ 4,8L d'eau déminéralisée, puis ajout d'environ 150 ml d'une solution de soude pour arriver à un pH de 2,4. La suspension est ensuite diluée avec de l'eau déminéralisée à un volume qui correspond au volume poreux du support alumine dont la surface spécifique est de 71 m$^2$/g mise en forme sous forme de billes. Cette solution est ensuite imprégnée en tonneau d'imprégnation à une vitesse de rotation de 14 tr/min et un débit d'ajout de la solution de 5,4l/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C3 ainsi préparé contient 0,19% poids de palladium par rapport au poids total de catalyseur.

La caractérisation du catalyseur C3 par microsonde de Castaing montre que 80% du Pd est réparti sur une croûte d'épaisseur environ 112 μm.

Le sodium est réparti de façon homogène avec un coefficient de répartition R(Na)=0,92.

La dispersion apparente du palladium du catalyseur C3 est de 23%.

### Exemple 4 : préparation d'un catalyseur C4 selon l'invention

Dans cet exemple, la surface spécifique du support est conforme à l'invention. Par ailleurs, l'étape d'imprégnation est conforme à l'invention.

Une suspension colloïdale d'oxyde de Pd est préparée sous agitation à 25°C par dilution de 230 g d'une solution de nitrate de palladium Pd(NO$_3$)$_2$ contenant 8,5 % poids de palladium Pd avec environ 4.8L d'eau déminéralisée, puis ajout d'environ 150 ml d'une solution de soude pour arriver à un pH de 2,4. La suspension est ensuite diluée avec de l'eau déminéralisée à un volume qui correspond au volume poreux du support alumine dont la surface spécifique est de 210 m$^2$/g mise en forme sous forme de billes. Cette solution est ensuite imprégnée en tonneau d'imprégnation à une vitesse de rotation de 14 tr/min et un débit d'ajout de la solution de 5,4l/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C4 ainsi préparé contient 0,19% poids de palladium par rapport au poids total de catalyseur.

La caractérisation du catalyseur C4 par microsonde de Castaing montre que 80% du Pd est réparti sur une croûte d'épaisseur environ 65 μm.

Le sodium est réparti de façon homogène avec un coefficient de répartition R(Na)=0,92.

La dispersion apparente du palladium du catalyseur C4 est de 27%.

### Exemple 5: préparation d'un catalyseur C5 selon l'invention

Dans cet exemple, la surface spécifique du support est conforme à l'invention. Par ailleurs, l'étape d'imprégnation est conforme à l'invention.

Une suspension colloïdale d'oxyde de Pd est préparée sous agitation à 25°C par dilution de 230 g d'une solution de nitrate de palladium Pd(NO$_3$)$_2$ contenant 8,5 % poids de palladium Pd avec environ 7,5L d'eau déminéralisée, puis ajout d'environ 150 ml d'une solution de soude pour arriver à un pH de 2,4. La suspension est ensuite diluée avec de l'eau déminéralisée à un volume qui correspond au volume poreux du support alumine dont la surface spécifique est de 140 m$^2$/g mise en forme sous forme de billes. Cette solution est ensuite imprégnée en tonneau d'imprégnation à une vitesse de rotation de 14 tr/min et un débit d'ajout de la solution de 5,4l/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C5 ainsi préparé contient 0,19% poids de palladium par rapport au poids total de catalyseur.

La caractérisation du catalyseur C5 par microsonde de Castaing montre que 80% du Pd est réparti sur une croûte d'épaisseur environ 93 μm.

Le sodium est réparti de façon homogène avec un coefficient de répartition R(Na)=0,92.

La dispersion apparente du palladium du catalyseur C5 est de 21%.

### Exemple 6 : préparation d'un catalyseur C6 selon l'invention

Dans cet exemple, la surface spécifique du support est conforme à l'invention. L'étape d'imprégnation du procédé est conforme à l'invention.

Une suspension colloïdale d'oxyde de Pd est préparée sous agitation à 25°C par dilution de 230 g d'une solution de nitrate de palladium $Pd(NO_3)_2$ contenant 8,5 % poids de palladium Pd avec environ 6,2L d'eau déminéralisée, puis ajout d'environ 150ml d'une solution de soude pour arriver à un pH de 2,4. La suspension est ensuite diluée avec de l'eau déminéralisée à un volume qui correspond au volume poreux du support alumine dont la surface spécifique est de 210 m$^2$/g mise en forme sous forme d'extrudés trilobés d'une longueur moyenne comprise entre 3 et 6 mm. Cette solution est ensuite imprégnée en tonneau d'imprégnation à une vitesse de rotation de 14tr/min et un débit d'ajout de la solution de 5,4l/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C6 ainsi préparé contient 0,19% poids de palladium par rapport au poids total de catalyseur.

La caractérisation du catalyseur C6 par microsonde de Castaing montre que 80% du Pd est réparti sur une croûte d'épaisseur environ 25 $\mu$m.

Le sodium est réparti de façon homogène avec un coefficient de répartition R(Na)=0,92.

La dispersion apparente du palladium du catalyseur C6 est de 31%.

### Exemple 7 : préparation d'un catalyseur C7 selon l'invention

Dans cet exemple, la surface spécifique du support est conforme à l'invention. L'étape d'imprégnation du procédé est conforme à l'invention.

Une suspension colloïdale d'oxyde de Pd est préparée sous agitation à 25°C par dilution de 230 g d'une solution de nitrate de palladium $Pd(NO_3)_2$ contenant 8,5 % poids de palladium Pd avec environ 6,2L d'eau déminéralisée, puis ajout d'environ 150ml d'une solution de soude pour arriver à un pH de 2,4. La suspension est ensuite diluée avec de l'eau déminéralisée à un volume qui correspond au volume poreux du support alumine dont la surface spécifique est de 200 m$^2$/g mise en forme sous forme d'extrudés cylindriques d'une longueur moyenne comprise entre 2 et 4 mm. Cette solution est ensuite imprégnée en tonneau d'imprégnation à une vitesse de rotation de 14tr/min et un débit d'ajout de la solution de 5,5l/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote. Le catalyseur est ensuite calciné sous un flux d'air 2 h à 450°C.

Le catalyseur C7 ainsi préparé contient 0,19% poids de palladium par rapport au poids total de catalyseur.

La caractérisation du catalyseur C7 par microsonde de Castaing montre que 80% du Pd est réparti sur une croûte d'épaisseur environ 29 $\mu$m.

Le sodium est réparti de façon homogène avec un coefficient de répartition R(Na)=0,92.

La dispersion apparente du palladium du catalyseur C7 est de 28%.

### Exemple 9 : préparation d'un catalyseur C8 (non conforme)

Dans cet exemple, la surface spécifique du support est conforme à l'invention. Cependant, l'étape d'imprégnation n'est pas conforme à l'invention.

Une suspension colloïdale d'oxyde de Pd est préparée sous agitation à 25°C par dilution de 230 g d'une solution de nitrate de palladium $Pd(NO_3)_2$ contenant 8,5 % poids de palladium Pd avec environ 6,2L d'eau déminéralisée, puis ajout d'environ 150ml d'une solution de soude pour arriver à un pH de 2,4. La suspension est ensuite diluée avec de l'eau déminéralisée à un volume qui correspond au volume poreux du support alumine dont la surface spécifique est de 210 m$^2$/g mise en forme sous forme d'extrudés cylindriques d'une longueur moyenne comprise entre 2 et 4 mm. Cette solution est ensuite imprégnée en tonneau d'imprégnation à une vitesse de rotation de 25 tr/min et un débit d'ajout de la solution de 15 L/h sur environ 10kg d'une alumine. Une étape de maturation du support imprégné avant séchage d'une durée de 20 h est effectuée sous air en milieu confiné et humide. Le solide obtenu est séché pendant 16h à 90°C sous débit d'azote.

Le catalyseur est ensuite calciné sous un flux d'air 2 h à 450°C.

Le catalyseur C8 ainsi préparé contient 0,19% poids de palladium par rapport au poids total de catalyseur.

La caractérisation du catalyseur C8 par microsonde de Castaing montre que 80% du Pd est réparti sur une croûte dont l'épaisseur est en dessous du seuil de détection.

Le sodium est réparti de façon homogène avec un coefficient de répartition R(Na)=0,91.

La dispersion apparente du palladium du catalyseur C8 est de 13%.

Les résultats des exemples 1 à 8 sont représentés dans le tableau 1 ci-après.

Tableau 1 : Caractéristiques techniques et morphologiques des catalyseurs C1 à C8

| | Vitesse de rotation (tr/min) | Débit ajout solution d'imprégnation (l/h) | $S_{BET}$ support $(m^2/g)$ | Forme | Taille croute $(\mu m)$ | Dispersion (%) |
|---|---|---|---|---|---|---|
| C1 (non conforme) | 6 | 22 | 71 | billes | 189 | 22 |
| C2 (non conforme) | 6 | 22 | 140 | billes | 150 | 26 |
| C3 (non conforme) | 14 | 5,4 | 71 | billes | 112 | 23 |
| C4 (conforme) | 14 | 5,4 | 210 | billes | 65 | 27 |
| C5 (conforme) | 14 | 5,4 | 140 | billes | 93 | 21 |
| C6 (conforme) | 14 | 5,4 | 210 | extrudés trilobés | 25 | 31 |
| C7 (conforme) | 14 | 5,5 | 200 | extrudés cylindriques | 29 | 28 |
| C8 (non conforme) | 25 | 15 | 210 | extrudés cylindriques | <sd* | 13 |
| *sd = seuil de détection | | | | | | |

**Exemple 9 : utilisation des catalyseurs C1 à C8 pour l'hydrogénation sélective de la coupe Pyqas vapocraquage**

Test catalytique en hydrogénation d'un mélange styrène isoprène en présence de S.

Avant le test catalytique, les catalyseurs C1 à C8 sont traités sous un flux de 1 litre d'hydrogène par heure et par gramme de catalyseur avec une montée en température de 300°C/h et un palier à 150°C pendant 2 heures.

Les catalyseurs sont ensuite soumis à un test d'hydrogénation dans un réacteur discontinu parfaitement agité de type « Grignard ». Pour ce faire, 4 ml de billes ou d'extrudés de catalyseur réduit sont fixées à l'abri de l'air dans un panier annulaire situé autour du mobile d'agitation. Les paniers utilisés dans les réacteurs sont de type Robinson Mahonnay.

L'hydrogénation est réalisée en phase liquide.

La composition de la charge est la suivante : 8% poids styrène, 8% poids isoprène, 10 ppm de S introduits sous forme de pentanethiol, 100 ppm de S introduits sous forme de thiophène, le solvant étant du n-heptane.

Le test est réalisé sous une pression constante de 3,5 MPa d'hydrogène et à une température de 45°C. Les produits de la réaction sont analysés par chromatographie en phase gazeuse.

Les activités catalytiques sont exprimées en moles de $H_2$ consommées par minute et par gramme de palladium et sont reportées dans le tableau 2 ci-après.

Tableau 2 : Activités mesurées en hydrogénation d'un mélange styrène-isoprène en présence de soufre

| Catalyseur | Activité* | %/Ref** |
|---|---|---|
| C1 (non conforme) | 0,31 | 41 |
| C2 (non conforme) | 0,63 | 84 |
| C3 (non conforme) | 0,52 | 69 |
| C4 (conforme) | 0,75 | 100 |
| C5 (conforme) | 0,73 | 97 |

(suite)

| Catalyseur | Activité* | %/Ref** |
|---|---|---|
| C6 (conforme) | 1,13 | 150 |
| C7 (conforme) | 0,97 | 130 |
| C8 (non conforme) | 0,59 | 79 |

\* en (moles $H_2$)/[min×(gramme de palladium)]
\*\* %/Ref correspond au gain converti en %, obtenu par rapport au catalyseur de référence C4 dont l'activité est défini à 100%.

On observe que les catalyseurs C4, C5, C6 et C7 ont une activité supérieure par rapport au catalyseurs C1, C2, C3 et C8 (non conformes à l'invention).

Le catalyseur C8 a une épaisseur de croûte trop fine (<20 $\mu$m) du fait de la haute surface spécifique de son support et du fait que le procédé de préparation est non adapté (la rotation du tonneau tournant est trop élevée, de même pour le débit un débit trop élevé également). Le catalyseur C1 ne donne pas des résultats satisfaisants en terme d'activité, ce dernier étant préparé par un procédé non conforme à l'invention. Par ailleurs le catalyseur C1 présente une surface spécifique trop faible.

Le catalyseur C2 ne donne pas de résultats satisfaisants en terme d'activité, ce dernier étant préparé par un procédé non conforme à l'invention.

Le catalyseur C3 ne donne pas des résultats satisfaisants en terme d'activité, quand bien même l'étape d'imprégnation de la solution colloïdale sur le support est conforme à l'invention, ce dernier étant préparé en utilisant un support dont la surface spécifique est trop faible.

## Revendications

1. Procédé de préparation d'un catalyseur comprenant du palladium, un support poreux comprenant au moins un oxyde réfractaire sélectionné dans le groupe constitué par la silice, l'alumine et la silice-alumine, ledit support poreux comprenant une surface spécifique comprise entre 140 et 250 m$^2$/g, telle que mesurée par la méthode B.E.T. selon la norme ASTM D3663-03, dans lequel au moins 80% poids du palladium est réparti dans une croûte à la périphérie dudit support, l'épaisseur de ladite croûte étant comprise entre 20 et 100 $\mu$m, la teneur en palladium dans le catalyseur est comprise entre 0,01 et 2 % poids par rapport au poids total du catalyseur, la dispersion métallique D du palladium est comprise entre 15% et 70%, lequel procédé comprenant les étapes suivantes :

   a) on prépare une solution colloïdale d'oxyde de palladium ou d'hydroxyde de palladium en phase aqueuse ;
   b) on ajoute à un débit compris 1 et 20 litre(s)/heure ladite solution obtenue à l'étape a) sur ledit support poreux ; ledit support poreux étant contenu dans un dispositif d'imprégnation rotatif fonctionnant à une vitesse de rotation comprise entre 4 et 20 tours/minute ;
   c) optionnellement, on soumet le support poreux imprégné obtenu à l'étape b) à une maturation afin d'obtenir un précurseur de catalyseur ;
   d) on sèche le précurseur de catalyseur obtenu à l'étape b) ou c) à une température comprise entre 70°C et 200°C pendant une durée comprise entre 0,5 et 20 heures ;
   e) on calcine le précurseur de catalyseur obtenu à l'étape d) à une température comprise entre 300°C et 500°C.

2. Procédé selon la revendication 1, **caractérisé en ce que** ledit support poreux se présente sous la forme d'une bille.

3. Procédé selon la revendication 1, **caractérisé en ce que** ledit support poreux se présente sous la forme d'un extrudé.

4. Procédé selon la revendication 3, **caractérisé en ce que** ledit support poreux se présente sous la forme d'un extrudé de longueur h comprise entre 2 et 10 mm.

5. Procédé selon les revendications 3 ou 4, **caractérisé en ce que** ledit support poreux comprend une section comportant au moins trois lobes.

6. Procédé selon la revendication 5, dans lequel le nombre de lobes de l'extrudé n est choisi dans le groupe constitué par les valeurs entières 3, 4, 5, 6, 7, 8, 9 et 10.

**7.** Procédé selon les revendications 5 ou 6, dans lequel le nombre de lobes n est choisi dans le groupe constitué par les valeurs entières 3 et 4.

**8.** Procédé selon la revendication 3, **caractérisé en ce que** l'extrudé se présente sous la forme d'un cylindre.

**9.** Procédé selon l'une quelconque des revendications précédentes, **caractérisé en ce que** ledit catalyseur comprend en outre de l'argent à une teneur comprise entre 0,02 et 3 % poids en argent par rapport au poids total du catalyseur.

**10.** Procédé selon l'une quelconque des revendications précédentes, **caractérisé en ce que** le support présente un diamètre de pores compris entre 2 et 50 nm.

**11.** Procédé selon l'une quelconque des revendications précédentes, dans lequel on réalise une étape c) de maturation pendant une durée comprise entre 0,5 et 40 heures.

**12.** Procédé selon l'une quelconque des revendications précédentes, dans lequel on soumet ledit catalyseur séché obtenu à l'issue de l'étape e) à un traitement réducteur par mise en contact avec un gaz réducteur à une température comprise entre 80 et 180°C.

**13.** Procédé selon l'une quelconque des revendications précédentes, dans lequel le dispositif d'imprégnation rotatif de l'étape b) est un tonneau tournant.

**Patentansprüche**

**1.** Verfahren zur Herstellung eines Katalysators, umfassend Palladium, einen porösen Träger, umfassend mindestens ein hitzebeständiges Oxid, ausgewählt in der Gruppe, die von Siliziumoxid, Aluminiumoxid und Siliziumoxid-Aluminiumoxid gebildet ist, wobei der poröse Träger eine spezifische Oberfläche zwischen 140 und 250 $m^2/g$, wie durch das B.E.T.-Verfahren nach der Norm ASTM D3663-03 gemessen, umfasst, wobei mindestens 80 Gew.-% des Palladiums in einer Kruste an der Peripherie des Trägers verteilt sind, wobei die Dicke der Kruste zwischen 20 und 100 $\mu$m beträgt, der Palladiumgehalt in dem Katalysator zwischen 0,01 und 2 Gew.-% bezogen auf das Gesamtgewicht des Katalysators beträgt, die Metalldispersion D des Palladiums zwischen 15 % und 70 % beträgt, wobei das Verfahren die folgenden Schritte umfasst:

a) Herstellen einer kolloidalen Lösung von Palladiumoxid und Palladiumhydroxid in wässriger Phase;
b) Hinzufügen in einer Menge zischen 1 und 20 Liter/Stunde der in Schritt a) erhaltenen Lösung auf den porösen Träger; wobei der poröse Träger in der rotierenden Imprägniervorrichtung enthalten ist, die mit einer Rotationsgeschwindigkeit zwischen 4 und 20 Umdrehungen/Minute funktioniert;
c) optional Unterziehen des in Schritt b) erhaltenen imprägnierten porösen Trägers einer Reifung, um eine Katalysator-Vorstufe zu erhalten;
d) Trocknen der in Schritt b) oder c) erhaltenen Katalysator-Vorstufe bei einer Temperatur zwischen 70 °C und 200 °C während einer Dauer zwischen 0,5 und 20 Stunden;
e) Kalzinieren der in Schritt d) erhaltenen Katalysator-Vorstufe bei einer Temperatur zwischen 300 °C und 500 °C.

**2.** Verfahren nach Anspruch 1, **dadurch gekennzeichnet, dass** der poröse Träger in Form einer Kugel vorhanden ist.

**3.** Verfahren nach Anspruch 1, **dadurch gekennzeichnet, dass** der poröse Träger in Form eines Extrudats vorhanden ist.

**4.** Verfahren nach Anspruch 3, **dadurch gekennzeichnet, dass** der poröse Träger in Form eines Extrudats mit einer Länge h zwischen 2 und 10 mm vorhanden ist.

**5.** Verfahren nach den Ansprüchen 3 oder 4, **dadurch gekennzeichnet, dass** der poröse Träger einen Abschnitt, umfassend mindestens drei Lappen, umfasst.

**6.** Verfahren nach Anspruch 5, bei dem die Anzahl von Lappen des Extrudats n in der Gruppe ausgewählt ist, die von den ganzen Zahlen 3, 4, 5, 6, 7, 8, 9 und 10 gebildet ist.

**7.** Verfahren nach den Ansprüchen 5 oder 6, bei dem die Anzahl von Lappen n in der Gruppe ausgewählt ist, die von den ganzen Zahlen 3 und 4 gebildet ist.

8. Verfahren nach Anspruch 3, **dadurch gekennzeichnet, dass** das Extrudat in Form eines Zylinders vorhanden ist.

9. Verfahren nach einem der vorhergehenden Ansprüche, **dadurch gekennzeichnet, dass** der Katalysator ferner Silber in einem Gehalt zwischen 0,02 und 3 Gew.-% Silber bezogen auf das Gesamtgewicht des Katalysators umfasst.

10. Verfahren nach einem der vorhergehenden Ansprüche, **dadurch gekennzeichnet, dass** der Träger einen Porendurchmesser zwischen 2 und 50 nm aufweist.

11. Verfahren nach einem der vorhergehenden Ansprüche, bei dem ein Schritt c) des Reifens während einer Dauer zwischen 0,5 und 40 Stunden durchgeführt wird.

12. Verfahren nach einem der vorhergehenden Schritte, bei dem der nach dem Schritt e) erhaltene getrocknete Katalysator einer Reduktionsbehandlung durch Kontaktnahme mit einem Reduktionsgas bei einer Temperatur zwischen 80 und 180 °C unterzogen wird.

13. Verfahren nach einem der vorhergehenden Ansprüche, bei dem die rotierende Imprägniervorrichtung aus Schritt b) ein rotierendes Fass ist.

**Claims**

1. A process for the preparation of a catalyst comprising palladium, a porous support comprising at least one refractory oxide selected from the group constituted by silica, alumina and silica-alumina, said porous support comprising a specific surface area in the range 140 to 250 $m^2$/g in accordance with ASTM standard D3663-03, in which at least 80% by weight of the palladium is distributed in a crust at the periphery of said support, the thickness of said crust being in the range 20 to 100 $\mu$m, the palladium content in the catalyst is in the range 0.01% to 2% by weight with respect to the total catalyst weight, the metallic dispersion D of the palladium is in the range 15% to 70%, said process comprising the following steps:

   a) preparing a colloidal solution of palladium oxide or palladium hydroxide in an aqueous phase;
   b) adding said solution obtained from step a) to said porous support at a flow rate in the range 4 to 20 litre(s)/hour; said porous support being contained in a rotary impregnation device functioning at a rotational speed in the range 10 to 20 rpm;
   c) optionally, submitting the impregnated porous support obtained from step b) to a maturation in order to obtain a catalyst precursor;
   d) drying the catalyst precursor obtained from step b) or c) at a temperature in the range 70°C to 200°C for a period in the range 0.5 to 20 hours;
   e) calcining the catalyst precursor obtained from step d) at a temperature in the range 300°C to 500°C.

2. The process as claimed in claim 1, **characterized in that** said porous support is in the form of a bead.

3. The process as claimed in claim 1, **characterized in that** said porous support is in the form of an extrudate.

4. The process as claimed in claim 3, **characterized in that** said porous support is in the form of an extrudate with a length h in the range 2 to 10 mm.

5. The process as claimed in claim 3 or claim 4, **characterized in that** said porous support comprises a section comprising at least three lobes.

6. The process as claimed in claim 5, in which the number of lobes of the extrudate, n, is selected from the group constituted by the integer values 3, 4, 5, 6, 7, 8, 9 and 10.

7. The process as claimed in claim 5 or claim 6, in which the number of lobes, n, is selected from the group constituted by the integer values 3 and 4.

8. The process as claimed in claim 3, **characterized in that** the extrudate is in the form of a cylinder.

9. The process as claimed in any one of the preceding claims, **characterized in that** said catalyst further comprises silver at a content in the range 0.02% to 3% by weight of silver with respect to the total catalyst weight.

10. The process as claimed in any one of the preceding claims, **characterized in that** the support has a pore diameter in the range 2 to 50 nm.

11. The process as claimed in any one of the preceding claims, in which a maturation step c) is carried out for a period in the range 0.5 to 40 hours.

12. The process as claimed in any one of the preceding claims, in which said dried catalyst obtained from step e) undergoes a reduction treatment by contact with a reducing gas at a temperature in the range 80°C to 180°C.

13. The process as claimed in any one of the preceding claims, in which the rotary impregnation device of step b) is a revolving drum.

**Figure 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2006025302 A
- FR 2922784
- FR 2968578

**Littérature non-brevet citée dans la description**

- **D.R. LIDE.** CRC Handbook of Chemistry and Physics. 2000
- **ROUQUEROL F. ; ROUQUEROL J. ; SINGH K.** Adsorption by Powders & Porous Solids: Principle, methodology and applications. Academic Press, 1999
- **R. VAN HARDEVELD ; F. HARTOG.** The statistics of surface atoms and surface sites on metal crystals. *Surface Science,* 1969, vol. 15, 189-230
- **SORBIER et al.** Measurement of palladium crust thickness on catalyst by EPMA. *Materials Science and Engineering,* 2012, vol. 32